# EUROPÄISCHE PATENTSCHRIFT

(11) **EP 1 878 464 B1**
(45) Veröffentlichungstag und Bekanntmachung des Hinweises auf die Patenterteilung: **19.10.2011**
(21) Anmeldenummer: 07011928.4
(22) Anmeldetag: 19.06.2007
(51) Int. Cl.: A61N 1/05

(54) **Implantierbare Elektrodenvorrichtung**
Implantable electrode device
Dispositif à électrodes implantable

(30) Priorität: 12.07.2006 DE 102006032240
(43) Veröffentlichungstag der Anmeldung: 16.01.2008
(73) Patentinhaber: Biotronik CRM Patent AG, 6341 Baar (CH)
(72) Erfinder: Bartels, Klaus, Dr., 10115 Berlin (DE); Geistert, Wolfgang, Dr., 79618 Rheinfelden (DE)
(74) Vertreter: Lindner-Vogt, Karin L.

(56) Entgegenhaltungen:
- WO-A-00/57949
- WO-A-02/078782
- WO-A-2006/023930
- US-A- 5 571 163
- US-A1- 2003 023 294
- US-A1- 2004 062 852
- US-A1- 2004 215 306
- US-A1- 2005 165 452

## Beschreibung

Die Erfindung betrifft eine implantierbare Elektrodenvorrichtung mit den im Oberbegriff des Patentanspruches 1 angegebenen Merkmalen. Insbesondere bezieht sich die Erfindung auf sogenannte ICD-Elektrodensonden für implantierbare Defibrillatoren und Kardioverter, die über mindestens eine Schockelektrode verfügen.

Diese Elektroden sind in der Regel offene Drahtwendel ausgebildet, wobei der Draht verschiedene Querschnitte, wie rund, flach, ballig, konvex, konkav und dergleichen aufweisen kann. Die Drahtwendel ist auf einem Trägerschlauch aufgewickelt, welcher als Kern des allgemein als Elektrodengrundkörper bezeichneten Trägers dient.

Die implantierbare Elektrodenvorrichtung selbst kann verschiedene Bauformen aufweisen. So sind aus dem Stand der Technik bekannt
- transvenös einführbare und in einer Herzkammer oder im Koronarsinus platzierbare Elektrodenvorrichtungen mit einem im Wesentlichen runden Querschnitt und einer oder mehreren zylinderförmigen Schockelektroden, oder
- interkostal platzierbare Elektrodenvorrichtungen mit einem im Wesentlichen runden Querschnitt und einer oder mehreren zylinderförmigen Schockelektroden, die auch auf mehreren fingerartigen Fortsätzen angeordnet sein können..

Solche Elektrodenvorrichtungen sind beispielsweise in den Druckschriften US 5,324,328 A, US 5,571,163 A und WO 02/22208 A2 beschrieben.

Zum Hintergrund der vorliegenden Erfindung ist festzuhalten, dass die Berührung zwischen Drahtwendel bzw. Drahtgeflecht der Schockelektrode einerseits und Körpergewebe andererseits zu Irritationen führen kann, die wiederum immunologische Reaktionen, wie Entzündungen oder Bindegewebswachstum hervorrufen können. Letzteres führt zum Einwachsen der Elektrode mit der Folge, dass diese im Bedarfsfall nur schwer wieder entfernt werden kann.

Befindet sich eine wendelförmige Schockelektrode im Blutstrom, so kann es hingegen zu Blutgerinnung und Thrombusbildung kommen. Besonders problematisch ist dabei die Situation einer Schockabgabe über die Elektrode, da dabei Koagulationen an den Hinterschneidungen, also an den dem Gewebe abgewandten Seiten der Drähte der Schockelektrode hervorgerufen werden können.

Zur Verhinderung oder Reduzierung der vorstehend erörterten Reaktionen sind eine Reihe von Maßnahmen aus dem Stand der Technik bekannt. So ist in den Druckschriften US 5,931,862 A, US 6,546,292 B1 und US 2003/0023294 A1 beschrieben worden, dass Einwachsen der Elektrodenvorrichtung durch Beschichtungen zum Beispiel mit einem PTFE-Material zu reduzieren. Derartige Beschichtungen weisen Poren auf, die einerseits zu klein sind, um Zellen einwachsen zu lassen, andererseits für Flüssigkeiten jedoch permeabel sind. So können Ionen die Beschichtung passieren und es wird ein Stromfluss ermöglicht. Nachteilig dabei ist jedoch, dass auf diese Weise die aktive Oberfläche der Elektrode deutlich reduziert wird und die Defibrillationsschwelle damit ansteigt. Problematisch ist ferner, dass sich solche Beschichtungen ablösen und als Fremdkörper in den Blutkreislauf gelangen können.

Die oben erwähnten negativen Reaktionen können ferner durch Medikamentengaben vermieden oder reduziert werden. Die US 4,506,680 A1 zeigt in diesem Zusammenhang eine Elektrodenvorrichtung mit einem Medikamentendepot an der Elektrodenspitze. Das Medikament ist dabei in einem Polymerpfropf untergebracht, der in einem Hohlraum in der Elektrodenspitze sitzt. Bei Beaufschlagung des Pfropfs mit Körperflüssigkeit quillt dieser auf und setzt den medizinischen Wirkstoff im Pfropf frei. Der Wirkstoff gelangt dann über eine poröse Beschichtungsmatrix auf der Elektrodenspitze in den Körper. Dieses Medikamentendepot ist dahingehend nachteilig, dass es einerseits ein separat zu montierendes Bauteil darstellt, andererseits sich relativ weit von der eigentlichen Schockelektrode an der Spitze entfernt befindet.

Aus der bereits oben erwähnten US 5,571,163 A und US 5,324,324 A sowie aus den Patentanmeldungen US 2004/0215306 A1, WO 2006/023930 A2 und WO 02/078782 A1 ist es bekannt, dass entzündungshemmende Medikamente durch eine Beschichtung der Elektrodenspitze und/oder der Defibrillationselektrode verabreicht werden. Die Medikamente sind dabei in ein Polymer eingebettet. Nachteil dieser Beschichtungen ist, dass sie einerseits eine relativ geringe Menge an medizinischem Wirkstoff enthalten und dass sie andererseits dieses Medikament nur über einen relativ kurzen Zeitraum abgeben, da sie sich relativ schnell auf- bzw. ablösen. Außerdem behindert auch hier wieder die Beschichtung der Defibrillationselektrode den Stromfluss erheblich, wodurch die Defibrillationsschwelle ansteigt.

Weitere Medikamentenanbindungen sind beispielsweise aus der WO 00/57949 bekannt. Diese Patentanmeldung zeigt eine Elektrodenvorrichtung mit einem Medikamentendepot. Dieses Depot ist jedoch innerhalb des Leitungslumens angebracht und wirkt lediglich auf die aktive Fixierungschraube, die bei der Befestigung aus dem distalen Ende der Elektrodenvorrichtung herausgeschraubt werden kann.
Insbesondere sei an dieser Stelle auf die US 2005/0165452 A1 verwiesen, aus der eine Elektrodenvorrichtung mit einer Schockelektrode bekannt ist, bei der die eingangs genannte Drahtwendel auf dem Trägerschlauch so gewickelt ist, dass zwischen den einzelnen Windungen der Drahtwendel helixförmige Zwischenräume freigelassen sind, die eine Medikamente enthaltende Polymerschicht aufweist. Aufgrund der Geringe dieses Medikamentenvolumens kann jedoch die Wirksamkeit der Medikamente enthaltenden Polymerschicht nicht über einen längeren Zeitraum sichergestellt werden.

Ausgehend von der geschilderten Problematik liegt der Erfindung die Aufgabe zugrunde, eine Elektrodenvorrichtung so zu verbessern, dass eine örtlich möglichst nahe der Elektrode erfolgende, gleichmäßige Medikamentenabgabe über einen möglichst langen Zeitraum erzielbar ist.

Diese Aufgabe wird durch die Merkmale des Anspruches 1 gelöst. Die Lösung zeichnet sich dadurch aus, dass der Trägerschlauch des Elektrodengrundkörpers im Bereich der Elektrode längs axial verlaufende Hohlkehlen aufweist, und sich die Medikamentendepot-Füllung aus biokompatiblem Polymermatrixmaterial in die Hohlkehlen erstreckt. Das Medikamentendepot ist durch diese Ausgestaltung quasi in die Elektrode integriert, indem zusätzlich zu den zwischen den einzelnen Windungen der Drahtwendel bestehenden kleine Lücken die Hohlkehlen zwischen Drahtwendel und Trägerschlauch mit einem flexiblen, das zu verabreichende Medikament tragenden Kunststoff gefüllt sind. Die Medikamentendepotfüllung dient somit gleichzeitig als Stabilisierung und Fixierung der Drahtwendel bei gleichzeitigem Erhalt der Flexibilität der Elektrode. Auch sind durch die Medikamentendepotfüllung die problematischen Hohlkehlen- und Zwischenbereiche der Elektrode aufgefüllt und damit nicht mehr schädlich.

Bevorzugtermaßen ist die Medikamentendepotfüllung auf der Basis eines flexiblen Kunststoffmaterials als Träger hergestellt, die einen darin fein verteilten, daraus freisetzbaren medizinischen Wirkstoff enthält.

Eine besonders körperverträgliche und wirksame Ausführungsform der Elektrodenvorrichtung liegt dann vor, wenn die Medikamentendepotfüllung den Raum zwischen den Windungen der Drahtwendel vollständig ausfüllt bzw. die Drahtwendel mit seiner Rückseite in die Medikamentendepotfüllung eingebettet ist. Die elektrisch aktive Oberfläche der Drahtwendel bleibt dabei einerseits praktisch vollständig erhalten, was die Defibrillationsschwelle niedrig hält. Andererseits ist die Drahtwendel optimal von Wirkstoff abgebender Materie umgeben, sodass eine gut dosierte Medikamentenabgabe in unmittelbarer Nähe der traumatisch problematischen Schockelektrode erzielt wird.

Bei dem Kunststoffmaterial der Medikamentendepotfüllung handelt es sich vorzugsweise um ein biokompatibles Polymer-Matrixmaterial, wie Silikon, Polyurethan oder ein Komposit aus diesen beiden Materialien.

Der medizinische Werkstoff kann beispielsweise ein Steroid, wie Dexamethason-Acetat, Dexamethason-Natrium-Phosphat oder Beclomethason und damit ein entzündungshemmender Wirkstoff, ein Heparin und damit gerinnungshemmender Wirkstoff, Sirolimus, Paclitaxel oder eine Magnesiumlegierung und damit antiproliferatives Medikament oder eine Kombination aus den vorstehenden Wirkstoffen sein.

Grundsätzlich ist das Trägerpolymer für die Medikamentendepotfüllung so formuliert, dass es hinreichend permeabel ist, sodass der untergemischte medizinische Wirkstoff mit der Zeit herausgelöst werden kann. Insbesondere bei Untermischung geringer Medikamentenmengen kann dieser Prozess dadurch unterstützt werden, dass die Medikamentendepotfüllung eine biodegradierbare Komponente, zum Beispiel ein Salz, ein Zucker, ein Polylactat oder ein Gel enthält. Löst sich diese Komponente im Körper auf, so werden mikroskopische Kanäle in der Medikamentendepotfüllung gebildet, durch die tiefer in der Polymerschicht liegende Wirkstoffanteile besser nach außen dringen und abgegeben werden.

Weitere Merkmale, Vorteile und Einzelheiten der Erfindung ergeben sich aus der nachfolgenden Beschreibung von Ausführungsbeispielen anhand der beigefügten Zeichnungen. Es zeigen:
- Figur 1: eine ausschnittsweise Seitenansicht einer ICD-Elektrodenvorrichtung,
- Figur 2: einen Querschnitt der Elektrodenvorrichtung gemäß Schnittlinie A-A nach Fig. 1,
- Figur 3: eine ausschnittsweise Draufsicht auf eine flächenhafte Defibrillationselektrode, und
- Figur 4: einen Schnitt entlang der Schnittlinie B-B gemäß Fig. 3 der Defibrillationselektrode.

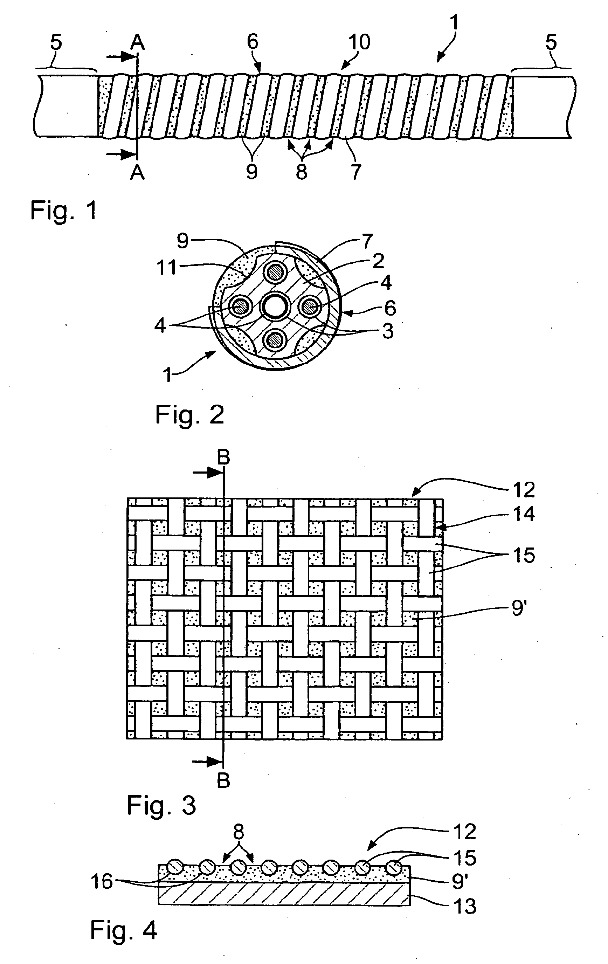

Die Figuren 1 und 2 zeigen eine im Herzen implantierbare ICD-Elektrodenvorrichtung, die einen langgestreckten, schlauchartigen Elektrodenkörper 1 aufweist. Dieser weist als Kern einen Trägerschlauch 2 auf, in dem mehrere Lumen 3 zur Durchführung der elektrischen Zuleitungen 4 für die in Figuren 1 und 2 nicht näher dargestellten Schock- und Stimulationselektroden der ICD-Elektrodenvorrichtung laufen.

Zwischen zwei äußeren Schlauchabschnitten 5 ist auf den Trägerschlauch 2 eine offene Drahtwendel 6 gewickelt, die aus außen konkav gewölbtem Flachdrahtmaterial 7 besteht. Zwischen den einzelnen Windungen der Drahtwendel 6 sind helixförmige Zwischenräume 8 freigelassen, die mit einer Medikamentdepotfüllung 9 vollständig ausgefüllt sind. An der Oberfläche des Elektrodenkörpers sind im Bereich der Schockelektrode 10 damit keine Hinterschneidungen vorhanden. Lediglich die balligen Außenseiten des Flachdrahtmaterials 7 stehen etwas erhaben vor.

Wie aus Fig. 2 deutlich wird, weist der Trägerschlauch 2 im Bereich der Schockelektrode 10 längs axial verlaufende Hohlkehlen 11 auf, in die sich die Medikamentendepotfüllung 9 hineinerstreckt.

Letztere besteht - wie bereits oben erwähnt - aus einem biokompatiblen Polymer-Matrixmaterial, wie beispielsweise Silikon, in das ein medizinischer Wirkstoff, wie beispielweise Heparin, eingebettet ist. Der Wirkstoff kann bei Beaufschlagung durch Körperflüssigkeit aus dem Kunststoffmaterial austreten und - im Falle von Heparin -gerinnungshemmende Wirkung entfalten.

Die Medikamentendepotfüllung 9 kann beispielsweise durch Untermischung des medizinischen Wirkstoffes in das flüssige Silikon-Trägermaterial und anschließendes Einbringen dieses Füllungsmaterials in die Zwischenräume 8 und die durch die Hohlkehlen 11 gebildeten Hinterschneidungen sowie anschließendes Aushärten des Füllungsmaterials gebildet werden.

Alternativ dazu kann die Medikamentendepotfüllung 9 durch Einbringen des flüssigen Trägermaterials in die Zwischenräume 8 und Hohlkehlen 11, Aushärten dieses Materials und Einbringen des medizinischen Wirkstoffes in Öffnungen des ausgehärteten Füllungsmaterials hergestellt werden. Letztere können bereits während des Aushärtens durch entsprechende Formgebung, durch eine mechanische Bearbeitung oder durch Herauslösen eines im Füllungsmaterial verteilten, löslichen Fremdmaterials gebildet sein. Der medizinische Wirkstoff kann dann durch Tauchen der Schockelektrode 10 mit dem ausgehärteten Füllungsmaterial in eine Medikamentenlösung oder -suspension oder durch Einsetzen von wirkstoffhaltigen Partikeln oder Fäden in die hergestellten Öffnungen eingebracht werden.

Die in den Figuren 3 und 4 gezeigte Einrichtung, die allerdings nicht Teil der Erfindung ist, stellt eine flächige Defibrillationselektrode 12 dar, bei der auf einem flexiblen, blattförmigen Träger 13 ein Drahtgeflecht 14 über die Medikamentendepotfüllung 9' festgelegt ist. Das Drahtgeflecht 14 ist aus gewebtem Runddrahtmaterial 15 gebildet, wobei die Zwischenräume 8 zwischen den sich kreuzenden Drähten und die unter den Drähten liegenden Hinterschneidungsbereiche 16 durch die Medikamentendepotfüllung 9' nicht nur geschlossen sind. Das Drahtgeflecht 14 ist vielmehr rückseitig in die Medikamentendepotfüllung 9' so eingebettet, dass Letztere die Fixierung des Drahtgeflechtes 14 am Träger 13 herstellt.

Für die Herstellung und den Aufbau der Medikamentendepotfüllung 9' gelten die im Zusammenhang mit Figuren 1 und 2 gemachten Ausführungen, sodass sich weitere Erläuterungen erübrigen.

## Patentansprüche

1. Implantierbare Elektrodenvorrichtung, umfassend
- einen Elektrodengrundkörper (1), welcher als Kern einen Trägerschlauch (2) umfasst, und
- mindestens eine daran angeordnete Elektrode (10), die aus einer offenen Drahtwendel (6) gebildet ist, wobei die Drahtwendel auf dem Trägerschlauch (2) so gewickelt ist, dass zwischen den einzelnen Windungen der Drahtwendel helixförmige Zwischenräume (8) freigelassen sind, welche eine Medikamentendepot-Füllung (9) aus biokompatiblem Polymermatrixmaterial aufweisen,
**dadurch gekennzeichnet, dass**
- der Trägerschlauch im Bereich der Elektrode längs axial verlaufende Hohlkehlen (11)aufweist,und
- sich die Medikamentendepot-Füllung (9) in die Hohlkehlen hinein erstreckt.

2. Elektrodenvorrichtung nach Anspruch 1, **dadurch gekennzeichnet, dass** die Medikamentendepot-Füllung (9) ein flexibles Kunststoffmaterial als Träger und einen darin verteilten, daraus freisetzbaren medizinischen Wirkstoff enthält.

3. Elektrodenvorrichtung nach Anspruch 1 oder 2, **dadurch gekennzeichnet, dass** die Medikamentendepot-Füllung (9) den Raum zwischen den Drahtwendeln (6) vollständig ausfüllt.

4. Elektrodenvorrichtung nach Anspruch 3, **dadurch gekennzeichnet, dass** die Drahtwendel (6) mit ihrer Rückseite in die Medikamentendepot-Füllung (9) eingebettet ist.

5. Elektrodenvorrichtung nach einem der vorgenannten Ansprüche, **dadurch gekennzeichnet, dass** das Kunststoffmaterial der Medikamentendepot-Füllung (9) ein biokompatibles Polymer-Matrixmaterial, insbesondere aus Silikon und/oder Polyurethan ist.

6. Elektrodenvorrichtung nach einem der vorgenannten Ansprüche, **dadurch gekennzeichnet, dass** der medizinische Wirkstoff in der Medikamentendepot-Füllung (9) entzündungshemmend, gerinnungshemmend und/oder antiproliferativ ist.

7. Elektrodenvorrichtung nach einem der vorgenannten Ansprüche, **dadurch gekennzeichnet, dass** die Medikamentendepot-Füllung (9) eine biodegradierbare Komponente enthält.

8. Elektrodenvorrichtung nach einem der vorgenannten Ansprüche, **dadurch gekennzeichnet, dass** die Medikamentendepot-Füllung (9) durch Untermischen eines medizinischen Wirkstoffes in ein flüssiges Trägermaterial, Einbringen dieses Füllungsmaterials in die Hohlkehlen (11) und/oder Zwischenräume (8) und Aushärten des Füllungsmaterials gebildet ist.

9. Elektrodenvorrichtung nach einem der vorgenannten Ansprüche, **dadurch gekennzeichnet, dass** die Medikamentendepot-Füllung (9) durch Einbringen eines flüssigen Trägermaterials in die Hohlkehlen (11) und/oder Zwischenräume (8), Aushärten des Füllungsmaterials und Einbringen des medizinischen Wirkstoffes in Öffnungen des ausgehärteten Füllungsmaterials gebildet ist.

10. Elektrodenvorrichtung nach Anspruch 9, **dadurch gekennzeichnet, dass** die Öffnungen des Füllungsmaterials durch Formgebung während des Aushärtens, durch mechanische Bearbeitung oder durch Herauslösen eines im Füllungsmaterial verteilten Fremdmaterials gebildet sind.

11. Elektrodenvorrichtung nach Anspruch 9 oder 10, **dadurch gekennzeichnet, dass** der medizinische Wirkstoff durch Tauchen des Füllungsmaterials in eine Medikamentenlösung oder -suspension oder durch Einsetzen von wirkstoffhaltigen Partikeln oder Fäden in die Öffnungen eingebracht ist.

## Claims

1. An implantable electrode device, comprising:
- an electrode base body (1) which comprises a carrier tube (2) as the core, and
- at least one electrode (10) disposed thereon, which is formed of an open wire helix (6), wherein the wire helix is wound onto the carrier tube (2) such that helical intermediate spaces (8) comprising a drug depot-filling (9) composed of biocompatible polymer matrix material are exposed between the individual windings of the wire helix,
**characterized in that**
- the carrier tube comprises axially extending channels (11) in the region along the electrode, and
- the drug depot-filling (9) extends into the channels.

2. The electrode device according to claim 1, **characterized in that** the drug depot-filling (9) contains a flexible plastic material as the carrier, and a medicinal active agent which is distributed therein and can be released therefrom.

3. The electrode device according to claim 1 or 2, **characterized in that** the drug depot-filling (9) completely fills the space between the wire helixes (6).

4. The electrode device according to claim 3, **characterized in that** the wire helix (6) is embedded via the back side thereof in the drug depot-filling (9).

5. The electrode device according to one of the preceding claims, **characterized in that** the plastic material of the drug depot-filling (9) is a biocompatible polymer matrix material, composed in particular of silicone and/or polyurethane.

6. The electrode device according to one of the preceding claims, **characterized in that** the medicinal active agent in the drug depot-filling (9) is anti-inflammatory, anti-coagulative, and/or anti-proliferative.

7. The electrode device according to one of the preceding claims, **characterized in that** the drug depot-filling (9) contains a biodegradable component.

8. The electrode device according to one of the preceding claims, **characterized in that** the drug depot-filling (9) is formed by intermixing a medicinal active agent in a liquid carrier material, placing this filling material into the channels (11) and/or intermediate spaces (8), and hardening the filling material.

9. The electrode device according to one of the preceding claims, **characterized in that** the drug depot-filling (9) is formed by placing a liquid filling material into the channels (11) and/or intermediate spaces (8), hardening the filling material, and placing the medicinal active agent into openings of the hardened filling material.

10. The electrode device according to claim 9, **characterized in that** the openings in the filling material are formed by shaping during hardening, by mechanical machining, or by dissolving away a foreign material distributed in the filling material.

11. The electrode device according to claim 9 or 10, **characterized in that** the medicinal active agent is placed into the openings by immersing the filling material into a drug solution or suspension, or by inserting particles or filaments containing active agent.

## Revendications

1. Dispositif à électrodes implantable, comprenant
- un corps de base d'électrode (1), comprenant un tube de support (2) en tant que noyau, et
- au moins une électrode (10) montée sur celui-ci (10), formée par un boudin métallique ouvert (6), le boudin métallique étant enroulé de telle façon sur le tube de support (2), que des espaces intermédiaires hélicoïdaux (8), comportant un remplissage de dépôt de médicament (9) constitué d'un matériau à matrice polymère, sont dégagés entre les différents enroulements du boudin métallique,
**caractérisé en ce que**
- le tube de support comporte des gorges creuses (11) s'étendant le long de l'axe dans la région de l'électrode,
- le remplissage de dépôt de médicament (9) s'étend dans les gorges creuses.

2. Dispositif à électrode selon la revendication 1, **caractérisé en ce que** le remplissage de dépôt de médicament (9) contient une matière plastique souple en tant que support, et une substance active médicale, répartie dans celle-ci et libérable de celle-ci.

3. Dispositif à électrode selon la revendication 1 ou 2, **caractérisé en ce que** le remplissage de dépôt de médicament (9) remplit complètement l'espace entre les boudins métalliques (6).

4. Dispositif à électrode selon la revendication 3, **caractérisé en ce que** le boudin métallique (6) est enrobé dans le remplissage de dépôt de médicament (9) par son côté arrière.

5. Dispositif à électrode selon l'une des revendications précédentes, **caractérisé en ce que** la matière plastique du remplissage de dépôt de médicament (9) est une matière à matrice polymère biocompatible, en particulier en silicone et/ou en polyuréthane.

6. Dispositif à électrode selon l'une des revendications précédentes, **caractérisé en ce que** la substance active médicale dans le remplissage de dépôt de médicament (9) est anti-inflammatoire, anticoagulante et/ou antiproliférative.

7. Dispositif à électrode selon l'une des revendications précédentes, **caractérisé en ce que** le remplissage de dépôt de médicament (9) contient un composant biodégradable.

8. Dispositif à électrode selon l'une des revendications précédentes, **caractérisé en ce que** le remplissage de dépôt de médicament (9) est formé par mélange d'une substance active médicale dans un matériau de support liquide, par intégration du matériau de remplissage dans les gorges creuses (11) et/ou les espaces intermédiaires (8), et par durcissement du matériau de remplissage.

9. Dispositif à électrode selon l'une des revendications précédentes, **caractérisé en ce que** le remplissage de dépôt de médicament (9) est formé par intégration d'un matériau de support liquide dans les gorges creuses (11) et/ou les espaces intermédiaires (8), par durcissement du matériau de remplissage et par introduction de la substance active médicale dans des ouvertures du matériau de remplissage durci.

10. Dispositif à électrode selon la revendication 9, **caractérisé en ce que** les ouvertures du matériau de remplissage sont formées pendant le durcissement, par un traitement mécanique ou par évacuation d'une matière étrangère répartie dans le matériau de remplissage.

11. Dispositif à électrode selon la revendication 9 ou 10, **caractérisé en ce que** la substance active médicale est introduite dans les ouvertures en plongeant le matériau de remplissage dans une solution ou suspension médicamenteuse, ou en intégrant des particules ou fils contenant la substance active.
